# EUROPEAN PATENT APPLICATION

(11) **EP 2 005 989 A2**
(43) Date of publication of application: **24.12.2008**
(21) Application number: 08104419.0
(22) Date of filing: 16.06.2008
(51) Int. Cl.: A61M 29/00

(54) **Disposable uterine dilator**

(30) Priority: 21.06.2007 IT VI20070180
(71) Applicant: Slatinac, Ms Slavica, 11060 Belgrado (RS)
(72) Inventor: Slatinac, Ms Slavica, 11060 Belgrado (RS)
(74) Representative: Bettello, Pietro

(57) **Abstract**

Herein described is a disposable uterine dilator, which comprises a cylindrical element (5), held within which is a fluid, in particular made up of distilled water, said fluid possibly being compressed by a piston (1) and thus discharged from said cylindrical element (5) in a slow and gradual manner ending up filling a structure made up of, in particular, a rod-shaped flexible element (11) which, filling up gradually, once previously inserted into the uterine cervix, is susceptible to enlarge under the dilatation effect due to the addition of a fluid into it, thus determining its dilatation and hence the dilatation of the uterine cervix into which it is inserted.

## Description

The present finding regards a disposable uterine dilator.

Very often, both in the field of gynaecology and in the field of surgery, doctors need to dilate the uterine cervix. Currently, this is attained by means of a plurality of graduated metal cones, referred to as "Hegar's dilators".

Unfortunately, these dilators are difficult to use and they are also very invasive, in that they are susceptible to damaging the cervix tissue in a more or less substantial manner. Sometimes, the abovementioned the cervix tissue is even lacerated by the abovementioned dilators and in some cases even the uterus ends up being punctured. Concerning this, it should be borne in mind that the problem is particularly serious considering the fact that nearly half of the women, in their life, are subjected to a gynaecological intervention regarding the uterus. This percentage is constantly growing, also due to the increase of the average life of women.

In order to explain the inherent complexity encountered when using these dilators, provided hereinafter is a brief description of how they are used.

First and foremost, before inserting the first dilator into the uterine cervix, the latter needs to be previously stabilised. This is usually obtained by using single toothed forceps, which are devices similar to scissors with two elements mutually countering each other at one of its ends. The forceps are arranged in such a manner that, when they are closed, their pointed tips can penetrate into the cervix at its front side, leaving the cervix entrance only accessible from beneath. The operator can thus insert a narrower dilator and then use the forceps to apply a contraction once said dilators have been inserted properly. Subsequently, wider dilators are inserted until the desired dilatation is obtained.

Such procedure might apparently seem quite simple, but complications are very frequent. As a matter of fact, the operator cannot see the channel directly and thus has to rely on his sense of touch to guide the tip of the various dilators into the cervix channel of the uterus. In addition, the operator also has to rely on his sense of touch to know when to stop pushing and when to withdraw the dilator before it literally passes through the head of the cervix. In most cases, this manoeuvre is not difficult. In fact, in case of non-expectant women in the age of reproduction the channel usually has a diameter of about 3-4 mm and therefore the dilators can be inserted quite easily.

However, some women, in particular those in menopause and those who have undergone surgical operations due to cervix diseases, have a very narrow and scarred cervix. Given that the pressure required to push the dilators through in such cases is higher, it is quite difficult to detect whether the cervix is actually being dilated or whether, on the contrary, a false channel is being created. Furthermore, the exertion of a necessarily greater pressure is susceptible to causing the forceps to tear the tissue, leading to the laceration of the cervix.

Given that this procedure is "blind", the best way for the operator to realise whether he has actually dilated the cervix or, vice versa, whether he has not perforated the rear wall of the uterus, is to continue pushing the dilator forward until he feels some resistance. However, during pregnancy, the uterine wall is much narrower and softer and thus more difficult to feel by touch.

A first objective of the present finding is to provide a device which can be used as a uterine cervix dilator capable of drastically reducing the possibility of causing damage or injuries to the patient, following complications during the dilatation procedure.

A further objective of the finding is that of providing a human cervix dilating device, easily useable by expert doctors and trainee doctors.

Further objective of the finding is that of providing a device of the type described above in which the dilatation of the cervix is controlled in an accurate manner. Furthermore, the finding aims at providing a device in which the performance of such dilatation can be carried out by an averagely expert doctor.

Lastly, further objective of the present invention is that of providing a device of the type described above which is simple both from a construction and from a functional point of view and which uses the existing technologies for its conception. Lastly, the device in question must be easily adapted to be sterilised.

According to the finding, this is attained by providing a device according to the characterising part of the main claim. Further characteristics and embodiments of the finding are outlined in the dependent claims.

Now, following is a detailed description of the finding with reference with one of its particular embodiments, provided for exemplifying and non-limiting purposes, with the help of the attached drawing, wherein;

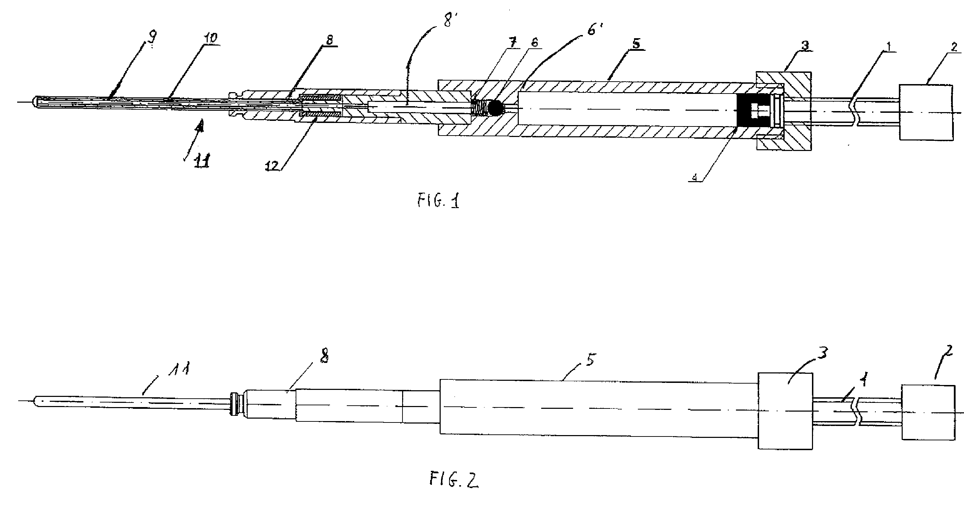

- Figures 1 and 2 respectively represent a sectional longitudinal view and a side view of the device subject of the finding.

As observable in the attached figures, the uterine dilator subject of the finding essentially consists in a device comprising an externally threaded piston 1. Such piston is susceptible to performing an axial movement, following the screwing action performed manually by the operator on the distal extension 2 of said piston into its seat, made up of a cylinder 5.

Present in the cylinder 5 is a liquid advantageously made up of distilled water. The end of the piston 1 arranged inside the cylinder 5 has a head 4, which serves both to exert pressure on the fluid held in the cylinder, and ensure the liquid-tightness against the leakage of the fluid from the cylinder itself. Present at the end of the cylinder into which the piston 1 penetrates is an internally threaded nut 3, whose threading allows obtaining a coupling by means screwing using the piston 1.

Vice versa, present at the end of the cylinder 5 opposite with respect to the end into which the piston 1 itself penetrates, is a check valve made up of a bead 6, pushed by a spring 7, which operates to keep said bead in contact with the entrance of a narrow channel 6', which communicates with the liquid holding internal part of the cylinder 5. In practice, when the head 4 is moved forward, in such a manner to compress the liquid, the check valve lets the liquid pass. Such liquid shall exceed the abovementioned check valve and reach the organs described hereinafter, while, should the opposite occur, the spring shall be "closed", preventing the fluid from flowing back into the cylinder 5.

Present downstream of the cylinder 5 is a rod-shaped flexible element 11, which, consists in a series or details "packed" one over the other, better described hereinafter and which, in combination, serve as a dilator balloon which dilates the uterus. Present between this rod-shaped element and the cylinder 5, is a communicating channel 8', which allows the inflow of the liquid into the internal part of the rod-shaped element 11. The channel is present inside a guide 8 (actually a cannula made of flexible material), which ensures sufficient perpendicular resistance of the rod-shaped element, required for the introduction of the same into the cervix, said guide 8 being integrally joined to a rod-shaped element 11, which starting from the centre of the same moves forward towards the external. The rod-shaped element 10 is coated by an elastic external membrane 9, adapted to receive and hold the water discharged through the piston 1 from the cylinder 5. Furthermore, said membrane 9 is in turn covered externally by a non-elastic tubular tissue 10, adapted to ensure resistance against water pressure which gradually increases inside the membrane 9. All the three elements 8, 9, 10 are mutually fixed by means of a bushing 12 arranged inside the guide 8.

Lastly, provided inside this rod-shaped flexible element 11 is an additional membrane, not illustrated for simplicity reasons, which, alongside holding all the "packed" elements compressed, being elastic in turn, it serves to facilitate the introduction of this rod-shaped flexible element 11 (and thus from the dilator balloon) into the uterine cervix, in that said membrane is made of silicone or similar material. As the amount water added into it increases, the dilator balloon is bound to fill up gradually, increasing its volume progressively. As previously observed, given that the movements of the piston 1 are necessarily slow and gradual, also the filling of the balloon (not illustrated in the figures for simplicity reasons) shall be slow and gradual, this allowing the operator to dilate the abovementioned balloon and thus also the cervix into which the deflated dilator balloon had been inserted previously, gradually overcoming in a uniform manner the resistance of the cervix itself. From a practical point of view, it has been ascertained that the maximum diameter to which the dilator balloon is inflated must be around 12.5-13 mm, which corresponds to the dimensions to which the cervix should be dilated to attain the objectives mentioned previously. Once the dilatation of the cervix has been obtained, the operator must wait for a few seconds until the cervix reaches such diameter. Then the balloon can be removed from the abovementioned cervix without requiring previous deflation of the same, given that the cervix tissue cannot be damaged, also due to the fact that element 11 is coated externally by a soft and thin elastic membrane. Clearly the check valve serves to ensure that the fluid remains inside the dilator balloon. Therefore, the abovementioned balloon can no longer be deflated and reused for obvious reasons of sterilisation. The device subject of the finding which can be easily sterilised, for example through gamma rays shall be delivered to the operator "ready to use", already having the distilled water inside it and enclosed in a transparent casing made of plastic material, just like the one used for normal injection syringes. All that the operator is required to do is open the sealed package, which protects the device already sterilised and thus go ahead to use it.

According to the points argued above, it can thus be observed that the device subject of the finding is capable of doubtlessly attaining the objectives outlined above, given that it is simple both from a construction point of view and from a functional point of view. The device can be used effortlessly even by an averagely expert personnel, (for example trainees or medical students) substantially reducing the possibility of causing unwanted damages to the patients on whom such operations are being performed.

## Claims

1. DISPOSABLE UTERINE DILATOR, **characterised in that** it comprises a cylinder (1), holding a liquid, operating on which are thrust and sealing means of the liquid itself (2, 3, 4), adapted to determine, following a thrust action performed on said liquid, its irreversible passage to a flexible guide (8) coaxial with the cylinder (5), said guide continuing with a rod-shaped flexible element (11) integrally joined to the same guide, the flexible element (11) being covered by a membrane (9), made of elastic material, adapted to receive and hold the liquid discharged from the cylinder (5), being inflated like a dilating balloon, capable of dilating the uterine cervix.

2. DILATOR, according to claim 1, **characterised in that** the means which determine the compression of the liquid held in the cylinder (5) are made up of an externally threaded piston (1), which advantageously has a distal extension (2) to facilitate its screwing action, said piston (1) being screwed into a threaded nut (3), provided at the entrance of the cylinder (5), the end of the piston present in the cylinder (5) being arranged in a head (4), which serves both to exert pressure onto the fluid held in the cylinder, and to ensure liquid-tightness against leakage of the fluid from the cylinder itself.

3. DILATOR, according to one or more of the preceding claims, **characterised in that** the cylinder (5) communicates with the elements arranged downstream of the abovementioned cylinder through a narrow channel (6'), arranged into which is a check valve, made up of a bead (6), pushed by a spring (7) which operates to keep said bead in contact with the entrance of said narrow channel (6'), which communicates with the internal part holding the liquid of the cylinder (5).

4. DILATOR, according to one or more of the preceding claims, **characterised in that** the membrane (9) is in turn covered by a non-elastic tubular tissue (10), adapted to ensure resistance against the pressure of the fluid which increases gradually in the membrane (9), the tubular element (10) being in turn covered by an additional membrane made of elastic material (for example silicone), adapted to keep the elements comprised in it compressed, as well as to facilitate the introduction of the structure into the uterine cervix.

5. DILATOR, according to one or more of the preceding claims **characterised in that** the dilator is sold enclosed in a transparent casing made of plastic material, suitably sterilised, for example by means of gamma rays.
